# FASCICULE DE BREVET EUROPEEN

(11) **EP 3 419 413 B1**
(45) Date de publication et mention de la délivrance du brevet: **18.12.2019**
(21) Numéro de dépôt: 17710344.7
(22) Date de dépôt: 22.02.2017
(51) Int. Cl.: C12M 1/34, C12N 1/12, A01G 33/00, C12M 1/00

(54) **PROCEDE DE CULTURE D'ORGANISMES PHOTOSYNTHETIQUES A L'AIDE D'UNE SOURCE DE CO2**
VERFAHREN ZUR ANZUCHT VON FOTOSYNTHETISCHEN ORGANISMEN UNTER VERWENDUNG EINER CO2-QUELLE
METHOD FOR THE CULTURE OF PHOTOSYNTHETIC ORGANISMS USING A CO2 SOURCE

(30) Priorité: 24.02.2016 FR 1651516
(43) Date de publication de la demande: 02.01.2019
(73) Titulaire: Université de Nantes, 44035 Nantes Cedex 1 (FR); Centre National de la Recherche Scientifique, 75794 Paris Cedex 16 (FR)
(72) Inventeur: PRUVOST, Jérémy, 44250 Saint Brevin Les Pins (FR); LE GOUIC, Benjamin, 46350 Guerande (FR)
(74) Mandataire: Bourgouin, André
(86) Numéro de dépôt international: PCT/FR2017/050393
(87) Numéro de publication internationale: WO 2017/144817

(56) Documents cités:
- WO-A1-2010/108049
- WO-A1-2012/056126
- WO-A1-2014/063229
- Espen Granum et al.: "A photobioreactor with pH control: Demonstration by growth of the marine diatom Skeletonema costatum", Journal of Plankton Research, vol. 24, no. 6 1 janvier 2002 (2002-01-01), pages 557-563, XP055319583, Extrait de l'Internet: URL:http://plankt.oxfordjournals.org/conte nt/24/6/557 [extrait le 2016-11-17]
- PATRICK J MCGINN ET AL: "Integration of microalgae cultivation with industrial waste remediation for biofuel and bioenergy production: opportunities and limitations", PHOTOSYNTHESIS RESEARCH ; OFFICIAL JOURNAL OF THE INTERNATIONAL SOCIETY OF PHOTOSYNTHESIS RESEARCH, SPRINGER, BERLIN, DE, vol. 109, no. 1 - 3, 9 mars 2011 (2011-03-09), pages 231-247, XP019952620, ISSN: 1573-5079, DOI: 10.1007/S11120-011-9638-0
- J. L. GARCÍA SÁNCHEZ ET AL: "Minimization of carbon losses in pilot-scale outdoor photobioreactors by model-based predictive control", BIOTECHNOLOGY AND BIOENGINEERING., vol. 84, no. 5, 24 septembre 2003 (2003-09-24), pages 533-543, XP055318340, US ISSN: 0006-3592, DOI: 10.1002/bit.10819
- M. BERENGUEL ET AL: "Model predictive control of pH in tubular photobioreactors", JOURNAL OF PROCESS CONTROL, vol. 14, no. 4, 1 juin 2004 (2004-06-01), pages 377-387, XP055318241, GB ISSN: 0959-1524, DOI: 10.1016/j.jprocont.2003.07.001
- HAN FEIFEI ET AL: "Enhanced lipid productivity of Chlorella pyrenoidosa through the culture strategy of semi-continuous cultivation with nitrogen limitation and pH control by CO2", BIORESOURCE TECHNOLOGY, ELSEVIER BV, GB, vol. 136, 14 mars 2013 (2013-03-14), pages 418-424, XP028591007, ISSN: 0960-8524, DOI: 10.1016/J.BIORTECH.2013.03.017

## Description

La présente invention a pour objet un procédé de culture d'organismes photosynthétiques à l'aide d'une source de CO₂, continue ou discontinue.

Les microalgues et les cyanobactéries sont des micro-organismes unicellulaires photosynthétiques pouvant produire différents types de matières organiques telles que les protéines, les glucides, les lipides et sont considérés comme des organismes optimaux pour obtenir des produits à haute valeur ajoutée, tels que des polysaccharides fonctionnels, des caroténoïdes, des vitamines, des acides gras insaturés, etc.

En ce qui concerne les macroalgues, leur valeur alimentaire rend leur culture particulièrement intéressante.

En outre, lors de la culture des microalgues, des cyanobactéries et des macroalgues, du dioxyde de carbone, qui est le facteur principal du réchauffement de la planète, est consommé, donc éliminé. Ces organismes photosynthétiques ont un temps de multiplication plus court que les plantes terrestres (et donc la quantité de dioxyde de carbone peut être efficacement réduite), grandissent rapidement dans un milieu pauvre, et peuvent directement utiliser les gaz de combustion provenant des centrales électriques ou des usines.

Afin d'éliminer efficacement le dioxyde de carbone tout en produisant des microalgues, des cyanobactéries et des macroalgues et/ou des composés d'intérêt avec des rendements élevés, il est nécessaire de disposer d'installations permettant de s'adapter aux sources intermittentes de dioxyde de carbone, car c'est sous cette forme que le dioxyde de carbone est traditionnellement rejeté dans l'atmosphère.

Actuellement, l'effluent gazeux est notamment injecté directement dans le système de culture où il est transféré à la phase liquide de culture. Lors des périodes d'arrêt de la source intermittente de production, la culture consomme le carbone dissous résiduel ce qui peut amener à une limitation de croissance si le carbone dissous devient insuffisant.

L'effluent gazeux peut également être injecté dans une solution liquide en amont du système pour former une solution carbonatée, servant ensuite à l'alimentation du système de culture. Cela permet de disposer d'une source de carbone dissous pendant les périodes d'arrêt. Néanmoins, la dissolution importante du carbone impose de travailler à un pH élevé incompatible avec la régulation pH du système de culture qui impose une source acide, la consommation du carbone dissous par la croissance microalgale basifiant le milieu.

Ainsi, un but de la présente invention est de fournir un procédé permettant la culture optimale d'organismes photosynthétiques avec une source intermittente de CO₂ gazeux.

Un autre but de la présente invention est de fournir un procédé permettant de concilier les besoins permanents des systèmes de culture d'organismes photosynthétiques en apport de carbone dissous et en régulation du pH, avec une source intermittente de CO₂ gazeux.

L'invention a par conséquent pour objet l'utilisation d'une première composition aqueuse ayant un pH supérieur à pH_{H} et d'une deuxième composition ayant un pH inférieur à pH_{B} pour la culture d'organismes photosynthétiques, choisis parmi les microalgues, les cyanobactéries et les macroalgues, dans un système de culture comprenant un milieu de culture,
dans laquelle :
- ladite première composition aqueuse ayant un pH supérieur à pH_{H} est obtenue par mise en contact de CO₂ produit par une source de CO₂, continue ou discontinue, d'une base, d'eau et éventuellement de tout ou partie des constituants d'un milieu de culture algal;
- la deuxième composition aqueuse ayant un pH inférieur à pH_{B} est obtenue par dissolution de CO₂ produit par ladite source dans de l'eau ou une solution aqueuse contenant tout ou partie des constituants d'un milieu de culture algal;
le pH dudit milieu de culture étant tel que :
- quand le pH dudit milieu de culture atteint une limite haute, pH_{H}, la deuxième composition aqueuse et/ou du CO₂ produit par ladite source sont ajoutés audit milieu de culture de manière à faire baisser le pH, de préférence jusqu'à ce que le pH dudit milieu de culture atteigne une limite basse, pH_{B};
- quand le pH dudit milieu de culture atteint ladite limite basse, pH_{B}, la première composition aqueuse est ajoutée audit milieu de culture de manière à faire monter le pH, de préférence jusqu'à ce que le pH dudit milieu de culture atteigne une valeur intermédiaire, pH_{I}, avec pH_{B}< pH_{I}< pH_{H}; et
- éventuellement, quand le pH dudit milieu de culture est inférieur à pH_{H}, en particulier à pH_{I}, la première composition aqueuse est ajoutée audit milieu de culture de manière à faire monter le pH, en particulier jusqu'à ce que le pH dudit milieu de culture atteigne la valeur intermédiaire pH_{I}.

De façon surprenante, l'utilisation selon la présente invention des deux compositions aqueuses permet d'assurer les besoins permanents des systèmes de culture d'organismes photosynthétiques en apport de carbone dissous, tout en permettant une régulation du pH, le tout étant possible même avec une source intermittente de CO₂ gazeux.

Par « microalgue», on entend une algue microscopique.

Par « cyanobactéries », on entend les bactéries de l'embranchement des *Cyanobacteria,* de la classe des *Cyanophyceae.*

Par « macroalgue », on entend une grande algue ou une algue géante, c'est-à-dire une algue plus grande qu'une microalgue.

Par « source de CO₂ », on entend une source produisant du CO₂, notamment sous forme gazeuse, ou une composition, notamment gazeuse, comprenant du CO₂, notamment sous forme gazeuse.

Cette source peut être continue, c'est-à-dire délivre le CO₂ avec un débit non nul, sur une période donnée, par exemple une heure, un jour ou une semaine.

En particulier, ce débit non nul est constant.

Alternativement, la source peut être discontinue, c'est-à-dire tantôt délivre le CO₂ avec un débit non nul, tantôt ne produit pas de CO₂, sur une période donnée, par exemple une heure, un jour ou une semaine.

Par « milieu de culture algal », on entend un milieu permettant la culture desdits organismes photosynthétiques.

Outre lesdits organismes photo synthétiques choisis parmi les microalgues, les cyanobactéries et les macroalgues, c'est-à-dire la biomasse algale, le milieu de culture comprend des constituants permettant la croissance desdits organismes photosynthétiques et/ou la production de molécules d'intérêt par lesdits organismes photosynthétiques.

Le milieu de culture est par exemple composé d'eau, d'éléments nutritifs pour les organismes photosynthétiques comme les sels minéraux - tels que nitrates, ammonium, phosphates, sulfates et métaux type fer, magnésium, etc...-, des ions carbonatés, voire des éléments organiques de type sucre.

Par « pH_{H} », on entend la limite haute du pH du milieu de culture. pH_{H} est notamment défini pour être compatible avec la physiologie de l'organisme photosynthétique mis en culture. Ainsi pH_{H} peut être une valeur au-delà de laquelle la croissance desdits organismes photosynthétiques et/ou la production de molécules d'intérêt par lesdits organismes photosynthétiques n'est plus optimale.

Par « pH_{B} », on entend la limite basse du pH du milieu de culture. pH_{B} est notamment défini pour être compatible avec la physiologie de l'organisme photosynthétique mis en culture. Ainsi pH_{B} peut être une valeur en-deçà de laquelle la croissance desdits organismes photo synthétiques et/ou la production de molécules d'intérêt par lesdits organismes photo synthétiques n'est plus optimale.

pH_{B} et pH_{H} encadrent pH_{I}, Cette valeur intermédiaire est en particulier le pH permettant la croissance optimale desdits organismes photosynthétiques et/ou la production optimale de molécules d'intérêt.

Par « première composition aqueuse », on entend une composition comprenant de l'eau, en particulier une solution aqueuse, ayant un pH supérieur à pH_{H}.

La première composition aqueuse comprend éventuellement tout ou partie des constituants d'un milieu de culture algal, tels que définis plus haut.

Cette première composition aqueuse comprend des ions carbonate et des ions bicarbonate, les ions carbonate et bicarbonate y étant notamment les espèces carbonatées majoritaires.

Le carbone inorganique dissous (CID) comprend notamment les ions carbonate, les ions bicarbonate et l'acide carbonique.

Dans la première composition aqueuse, le CID est majoritairement sous forme d'ions carbonate et d'ions bicarbonate.

Ladite base permet d'augmenter le pH de la première composition aqueuse et la concentration en CID.

Par exemple, dans le cas d'une fumée industrielle comprenant 9% de CO₂, à 25°C, pH et CID sont liés comme indiqué dans le tableau suivant :

| **pH** | **CID (mM)** |
|---|---|
| 7,5 | 46,7 |
| 8 | 141,2 |
| 8,5 | 444,1 |
| 9 | 1441,2 |

Par « deuxième composition aqueuse », on entend une composition comprenant de l'eau, en particulier une solution aqueuse, ayant un pH inférieur à pH_{B}.

La deuxième, composition aqueuse comprend éventuellement tout ou partie des constituants d'un milieu de culture algal, tels que définis plus haut.

Cette deuxième composition aqueuse comprend de l'acide carbonique, l'acide carbonique y étant notamment l'espèce carbonatée majoritaire.

Dans la deuxième composition aqueuse, le CID est majoritairement sous forme d'acide carbonique.

Par exemple, dans le cas d'une fumée industrielle comprenant 9% de CO₂, à 25°C, le pH de la deuxième composition aqueuse, à l'équilibre, est de 4,42 et la concentration en CID de 3mM.

Selon un mode de réalisation avantageux, l'invention concerne une utilisation telle que définie précédemment, dans laquelle les organismes photosynthétiques sont choisis dans le groupe constitué :
- des microalgues, en particulier des microalgues des genres *Chlorella, Nannochloropsis, Chlamydomonas, Tetraselmis, Scendesmus, Parachlorella, Porphyridium; Botryococcus* et *Neochloris* ;
- des cyanobactéries, en particulier des genres *Arthrospira, Aphazomenon et Synechocystis;* et
- des macroalgues, en particulier les macroalgues Ulva, Fucus, Palmaria.

Le genre de cyanobactéries *Arthrospira* est communément appelé spiruline.

Selon un mode de réalisation avantageux, l'invention concerne une utilisation telle que définie précédemment, dans laquelle la source de CO₂ est constituée de fumées industrielles, ladite la source de CO₂ étant en particulier choisie dans le groupe constitué des émissions de chaudière, de centrale thermique, de cimenterie, de sidérurgie, de raffinerie, d'usine de fabrication d'ammoniac, des procédés de fermentation et des procédés de digestion anaérobie.

Selon un mode de réalisation avantageux, l'invention concerne une utilisation telle que définie précédemment, dans laquelle la valeur de pH_{I} dans le milieu de culture est comprise de 6 à 10.

Selon un mode de réalisation avantageux, l'invention concerne une utilisation telle que définie précédemment, dans laquelle pH_{I} est le pH optimal de croissance desdits organismes photosynthétiques dans le milieu de culture.

Selon un mode de réalisation avantageux, l'invention concerne une utilisation telle que définie précédemment, dans laquelle pH_{H} dans le milieu de culture est tel que pH_{H} = pH_{I} + x, x étant compris de 0,02 à 1,5, en particulier de 0,1 à 0,2.

Selon un mode de réalisation avantageux, l'invention concerne une utilisation telle que définie précédemment, dans laquelle pH_{B} dans le milieu de culture est tel que pH_{B} = pH_{I} - y, y étant compris de 0,02 à 1,5, en particulier de 0,1 à 0,2.

Selon un mode de réalisation avantageux, l'invention concerne une utilisation telle que définie précédemment, dans laquelle ladite base est choisie dans le groupe constitué de l'hydroxyde de sodium et de l'hydroxyde de potassium.

Selon un mode de réalisation avantageux, l'invention concerne une utilisation telle que définie précédemment, dans laquelle la température du milieu de culture est comprise de 15°C à 35°C.

Selon un mode de réalisation avantageux, l'invention concerne une utilisation telle que définie précédemment, dans laquelle le système de culture est un système fermé.

Par « système de culture fermé », on entend une enceinte de culture isolée de son environnement extérieur par, par exemple, un matériau transparent afin de laisser passer la lumière. Ce système permet de mieux contrôler les conditions de culture et en particulier les apports de carbone, et d'éviter des contaminations extérieures par d'autres organismes. Cela amène au final à une productivité plus élevée d'une biomasse et de meilleure qualité.

Selon un mode de réalisation avantageux, l'invention concerne une utilisation telle que définie précédemment, dans laquelle le système de culture est un système ouvert.

Par « système de culture ouvert », on entend un système de culture à l'ambiant. L'intérêt est d'avoir un système moins couteux, par comparaison aux systèmes fermés.

L'invention concerne également un procédé de culture d'organismes photosynthétiques, choisis parmi les microalgues, les cyanobactéries et les macroalgues, à l'aide d'une source de CO₂, continue ou discontinue, dans lequel le CO₂ est dirigé au moyen de conduites et de vannes commandées de préférence par un automate :
- dans un système de culture comprenant un milieu de culture d'organismes photosynthétiques; et/ou
- dans de l'eau ou une solution aqueuse contenant tout ou partie des constituants d'un milieu de culture algal, pour obtenir en présence d'une base une première composition aqueuse ayant un pH supérieur à pH_{H}; et/ou
- dans de l'eau ou une solution aqueuse contenant tout ou partie des constituants d'un milieu de culture algal, pour obtenir une deuxième composition aqueuse ayant un pH inférieur à pH_{B}, par dissolution de CO₂ produit par ladite source dans l'eau ;
procédé caractérisé en ce que, lors de la culture d'organismes photosynthétiques dans ledit milieu de culture pour obtenir une biomasse d'organismes photosynthétiques à l'aide du CO₂ provenant de ladite source et/ou des ions carbonate et de l'acide carbonique respectivement contenus dans les première et deuxième compositions aqueuses:
i. quand le pH dudit milieu de culture atteint une limite haute, pH_{H}, la deuxième composition aqueuse et/ou du CO₂ produit par ladite source sont ajoutés audit milieu de culture de manière à faire baisser le pH, de préférence jusqu'à ce que le pH dudit milieu de culture atteigne une limite basse, pH_{B};
ii. quand le pH dudit milieu de culture atteint ladite limite basse, pH_{B}, la première composition aqueuse est ajoutée audit milieu de culture de manière à faire monter le pH, de préférence jusqu'à ce que le pH dudit milieu de culture atteigne une valeur intermédiaire, pH_{I}, avec pH_{B}< pH_{I}< pH_{H}; et
iii. éventuellement, quand le pH dudit milieu de culture est inférieur à pH_{H}, en particulier à pH_{I}, la première composition aqueuse est ajoutée audit milieu de culture, de manière à faire monter le pH, en particulier jusqu'à ce que le pH dudit milieu de culture atteigne la valeur intermédiaire pH_{I}.

De façon générale, la consommation du carbone dissous dans le milieu de culture par les organismes photosynthétiques en croissance fait augmenter le pH dudit milieu de culture.

Par exemple, en considérant un milieu de culture étant à un moment donné à pH_{I}, le pH va augmenter au cours de la croissance des organismes photosynthétiques. Lorsque le pH aura atteint pH_{H}, l'action i sera effectuée jusqu'à pH_{B}. Puis, l'étape ii sera effectuée, de préférence jusqu'à pH_{I}, cet enchaînement pouvant être renouvelé autant que nécessaire.

L'action iii peut être effectuée dans le cas suivant : lorsque le pH du milieu de culture est compris entre pH_{B} et pH_{I}, l'action iii peut être effectuée, de préférence jusqu'à pH_{I}. Le pH va alors augmenter au cours de la croissance des organismes photosynthétiques jusqu'à pH_{H}. L'action i sera alors effectuée jusqu'à pH_{B}. Puis, l'étape ii sera effectuée, de préférence jusqu'à pH_{I}, cet enchaînement pouvant être renouvelé autant que nécessaire.

On peut également, au lieu d'attendre que le pH du milieu augmente jusqu'à pH_{H} du fait la consommation du carbone dissous dans le milieu de culture par les organismes photosynthétiques en croissance, effectuer l'action iii.

Par exemple, en considérant un milieu de culture étant à un moment donné à pH_{I}, on peut effectuer l'action iii jusqu'à pH_{H}, puis enchainer l'étape i jusqu'à pH_{B} puis l'étape ii jusqu'à pH_{I}, et ainsi de suite.

Selon un mode de réalisation avantageux, l'invention concerne un procédé tel que défini précédemment, dans lequel les organismes photosynthétiques sont choisis dans le groupe constitué :
- des microalgues, en particulier des microalgues des genres *Chlorella, Nannochloropsis, Chlamydomonas, Tetraselmis, Scendesmus, Parachlorella, Porphyridium, Botryococcus* et *Neochloris;*
- des cyanobactéries, en particulier des genres *Arthrospira, Aphazomenon* et *Synechocystis;* et
- des macroalgues, en particulier les macroalgues Ulva, Fucus, Palmaria.

Selon un mode de réalisation avantageux, l'invention concerne un procédé tel que défini précédemment, dans lequel la source de CO₂ est constituée de fumées industrielles, ladite source de CO₂ étant en particulier choisie dans le groupe constitué des émissions de chaudière, de centrale thermique, de cimenterie, de sidérurgie, de raffinerie, d'usine de fabrication d'ammoniac, des procédés de fermentation et des procédés de digestion anaérobie.

Selon un mode de réalisation avantageux, l'invention concerne un procédé tel que défini précédemment, dans lequel la source de CO₂ est discontinue, et l'automate agit sur les différentes vannes reliant entres eux un système de culture d'organismes photosynthétiques comprenant ledit milieu de culture, la première composition aqueuse ayant un pH supérieur à pH_{H} et la deuxième composition ayant un pH inférieur à pH_{B} de telle manière que :
- lorsque la source de CO₂ produit du CO₂ :
   ∘ du CO₂ est dirigé dans de l'eau ou une solution aqueuse contenant tout ou partie des constituants d'un milieu de culture algal, pour obtenir en présence d'une base, ladite première composition aqueuse, et dans de l'eau ou une solution aqueuse contenant tout ou partie des constituants d'un milieu de culture algal pour obtenir ladite deuxième composition aqueuse par dissolution de CO₂ produit par ladite source dans l'eau ou la solution aqueuse ;
   ∘ (si désiré) du CO₂ est dirigé directement dans le système de culture si le pH est tel que pH_{B}< pH< pH_{H};
   ∘ quand le pH dudit milieu de culture atteint une limite haute, pH_{H}, du CO₂ produit par ladite source et éventuellement la deuxième composition aqueuse sont ajoutés audit milieu de culture de manière à faire baisser le pH, de préférence jusqu'à ce que le pH dudit milieu de culture atteigne une limite basse, pH_{B};
   ∘ quand le pH dudit milieu de culture atteint ladite limite basse, pH_{B}, le CO₂ produit par ladite source n'est plus ajouté audit milieu de culture, et si désiré, la première composition aqueuse est ajoutée audit milieu de culture de manière à faire monter le pH, de préférence jusqu'à ce que le pH dudit milieu de culture atteigne une valeur intermédiaire, pH_{I}. avec pH_{B}< pH_{I}< pH_{H};
- lorsque la source de CO₂ ne produit pas de CO₂ :
   ∘ la première composition aqueuse est ajoutée au milieu de culture de manière à apporter du carbone dissous dans ledit milieu de culture, provoquant une augmentation du pH jusqu'à ce que de préférence le pH dudit milieu de culture atteigne une valeur intermédiaire, pH_{I}, avec pH_{B}< pH_{I}< pH_{H};
   ∘ quand le pH dudit milieu de culture atteint une limite haute, pH_{H}, la deuxième composition aqueuse est ajoutée audit milieu de culture de manière à faire baisser le pH, de préférence jusqu'à ce que le pH dudit milieu de culture atteigne une limite basse, pH_{B.}

Les deux actions décrites lorsque la source de CO₂ ne produit pas de CO₂ peuvent être inversées. En d'autres termes, il n'y a pas d'ordre défini dans le cas général. De plus, ces actions peuvent être répétées autant que nécessaire.

Selon un mode de réalisation avantageux, lorsque la source de CO₂ ne produit pas de CO₂:
∘ la première composition aqueuse est ajoutée au milieu de culture de manière à apporter du carbone dissous dans ledit milieu de culture, provoquant une augmentation du pH jusqu'à ce que de préférence le pH dudit milieu de culture atteigne une valeur intermédiaire, pH_{I}, avec pH_{B}< pH_{I}< pH_{H}; puis
∘ quand le pH dudit milieu de culture atteint une limite haute, pH_{H}, la deuxième composition aqueuse est ajoutée audit milieu de culture de manière à faire baisser le pH, de préférence jusqu'à ce que le pH dudit milieu de culture atteigne une limite basse, pH_{B}.

En particulier; entre les deux actions mentionnées ci-dessus, le pH augmente, en particulier de pH_{I}, jusqu'à pH_{H} de part la consommation du carbone dissous dans le milieu de culture par les organismes photosynthétiques en croissance.

Selon un mode de réalisation avantageux, lorsque la source de CO₂ ne produit pas de CO₂:
∘ quand le pH dudit milieu de culture atteint une limite haute, pH_{H}, la deuxième composition aqueuse est ajoutée audit milieu de culture de manière à faire baisser le pH, de préférence jusqu'à ce que le pH dudit milieu de culture atteigne une limite basse, pH_{B}; puis
∘ la première composition aqueuse est ajoutée au milieu de culture de manière à apporter du carbone dissous dans ledit milieu de culture, provoquant une augmentation du pH jusqu'à ce que de préférence le pH dudit milieu de culture atteigne une valeur intermédiaire, pH_{I}, avec pH_{B}< pH_{I}< pH_{H}.

En particulier, le pH augmente avant la première action mentionnée ci-dessus, jusqu'à pH_{H} du fait de la consommation du carbone dissous dans le milieu de culture par les organismes photosynthétiques en croissance. Les première et deuxième actions s'enchaînent ensuite.

Selon un mode de réalisation avantageux, l'invention concerne un procédé tel que défini précédemment, dans lequel la valeur de pH_{I} dans le milieu de culture est comprise de 6 à 10.

Selon un mode de réalisation avantageux, l'invention concerne un procédé tel que défini précédemment, dans lequel pH_{I} est le pH optimal de croissance desdits organismes photosynthétiques dans le milieu de culture.

Selon un mode de réalisation avantageux, l'invention concerne un procédé tel que défini précédemment, dans lequel pH_{H} dans le milieu de culture est tel que pH_{H} = pH_{I}, + x, x étant compris de 0,02 à 1,5, en particulier de 0,1 à 0,2.

Selon un mode de réalisation avantageux, l'invention concerne un procédé tel que défini précédemment, dans lequel pH_{B} dans le milieu de culture est tel que pH_{B} = pH_{I} - y, y étant compris de 0,02 à 1,5, en particulier de 0,1 à 0,2.

Selon un mode de réalisation avantageux, l'invention concerne un procédé tel que défini précédemment, dans lequel ladite base est choisie dans le groupe constitué de l'hydroxyde de sodium et de l'hydroxyde de potassium.

Selon un mode de réalisation, le pH de la première composition aqueuse peut être compris de 7,5 à 9.

Selon un mode de réalisation, la concentration en CID dans la première composition aqueuse peut être comprise de 45 à 1450 mM.

Selon un mode de réalisation, le pH de la deuxième composition aqueuse, à l'équilibre, peut être compris de 4 à 5, et est en particulier d'environ 4,4.

Selon un mode de réalisation, la concentration en acide carbonique dans la deuxième composition aqueuse peut être comprise de 1 à 5 mM, et est en particulier d'environ 3mM.

Selon un mode de réalisation avantageux, l'invention concerne un procédé tel que défini précédemment, dans lequel la température du milieu de culture est comprise de 15°C à 35°C.

Selon un mode de réalisation avantageux, l'invention concerne un procédé tel que défini précédemment, dans lequel le système de culture est un système fermé.

Par « système de culture fermé », on entend une enceinte de culture isolée de son environnement extérieur par, par exemple, un matériau transparent afin de laisser passer la lumière. Ce système permet de mieux contrôler les conditions de culture et en particulier les apports de carbone, et d'éviter des contaminations extérieures par d'autres organismes. Cela amène au final à une productivité plus élevée d'une biomasse et de meilleure qualité.

Selon un mode de réalisation avantageux, l'invention concerne un procédé selon tel que défini précédemment, dans lequel le système de culture est un système ouvert.

Par « système de culture ouvert », on entend un système de culture à l'ambiant. L'intérêt est d'avoir un système moins couteux, par comparaison aux systèmes fermés.

L'invention concerne également un dispositif pour la culture d'organismes photosynthétiques, choisis parmi les microalgues, les cyanobactéries et les macroalgues, à l'aide d'une source de CO₂, continue ou discontinue, comprenant les éléments suivants:
- un moyen A de captation du CO₂ produit par ladite source, moyen comprenant de l'eau ou une solution aqueuse contenant tout ou partie des constituants d'un milieu de culture algal, et une base, et permettant l'obtention d'une première composition aqueuse ayant un pH supérieur à pH_{H};
- un moyen B de captation du CO₂ produit par ladite source, moyen comprenant de l'eau ou une solution aqueuse contenant tout ou partie des constituants d'un milieu de culture algal, et permettant l'obtention d'une deuxième composition aqueuse ayant un pH inférieur à pH_{B};
- un système de culture desdits organismes photosynthétiques, équipé de moyens de mesure du pH;
- des conduites et des vannes reliant entre eux la source de CO₂, le système de culture desdits organismes photosynthétiques et les moyens A et B
- un système dé commande et de contrôle des flux gazeux et liquides entre la source de CO₂, le système de culture desdits organismes photo synthétiques et les moyens A et B, de préférence un automate, caractérisé en que le système de commande est agencé de façon à ce que:
   ∘ le CO₂ produit par ladite source est dirigé vers ledit système de culture, et/ou lesdits moyens A et B de captation du CO₂;
   ∘ quand le pH dudit milieu de culture atteint une limite haute, pH_{H}, la deuxième composition aqueuse provenant du moyen B et/ou du CO₂ produit par ladite source sont ajoutés audit milieu de culture de manière à faire baisser le pH, de préférence jusqu'à ce que le pH dudit milieu de culture atteigne une limite basse, pH_{B};
   ∘ quand le pH dudit milieu de culture atteint ladite limite basse, pH_{B}, la première composition aqueuse provenant du moyen A est ajoutée audit milieu de culture de manière à faire monter le pH, de préférence jusqu'à ce que le pH dudit milieu de culture atteigne une valeur intermédiaire, pH_{I}, avec pH_{B}< pH_{I}< pH_{H}; et
   ∘ éventuellement, quand le pH dudit milieu de culture est inférieur à pH_{H}, en particulier à pH_{I}, la première composition aqueuse est ajoutée audit milieu de culture de manière à faire monter le pH, en particulier jusqu'à ce que le pH dudit milieu de culture atteigne la valeur intermédiaire pH_{I}.

Le moyen A est par exemple une cuve de carbonatation, comprenant de l'eau ou une solution aqueuse contenant tout ou partie des constituants d'un milieu de culture algal, et une base, dans laquelle du CO₂ est mis en contact avec l'eau ou la solution aqueuse, et la base, en particulier en faisant buller le CO₂ dans l'eau ou la solution aqueuse préalablement mis en contact avec la base.

Le moyen B est par exemple une cuve d'acidification, comprenant de l'eau ou une solution aqueuse contenant tout ou partie des constituants d'un milieu de culture algal, dans laquelle du CO₂ est mis en contact avec l'eau ou la solution aqueuse, en particulier en faisant buller le CO₂ dans l'eau ou la solution aqueuse.

Selon un mode de réalisation avantageux, l'invention concerne un dispositif tel que défini précédemment, dans lequel le système de culture est un système fermé.

Par « système de culture fermé », on entend une enceinte de culture isolée de son environnement extérieur par, par exemple, un matériau transparent afin de laisser passer la lumière. Ce système permet de mieux contrôler les conditions de culture et en particulier les apports de carbone, et d'éviter des contaminations extérieures par d'autres organismes. Cela amène au final à une productivité plus élevée d'une biomasse et de meilleure qualité.

Selon un mode de réalisation avantageux, l'invention concerne un dispositif tel que défini précédemment, dans lequel le système de culture est un système ouvert.

Par « système de culture ouvert », on entend un système de culture à l'ambiant. L'intérêt est d'avoir un système moins couteux, par comparaison aux systèmes fermés.

Selon un mode de réalisation avantageux, l'invention concerne un dispositif tel que défini précédemment, dans lequel le moyens A est équipé d'une sonde de pH et/ou de température,

Selon un mode de réalisation avantageux, l'invention concerne un dispositif tel que défini précédemment, dans lequel le moyen A est équipé d'un capteur de pression et d'un déverseur de pression.

Selon un mode de réalisation avantageux, l'invention concerne un dispositif tel que défini précédemment, dans lequel le moyen A est équipé d'un système permettant la formation de bulles, en particulier de bulles de taille contrôlée, plus particulièrement de bulles d'une-taille moyenne comprise de 10µm à 50µm, à partir du CO₂ produit par la source de CO₂.

La formation de petites bulles, de l'ordre du micromètre présente l'avantage d'augmenter la surface de contact avec le moyen A et ainsi d'améliorer le transfert du CO2.

Selon un mode de réalisation avantageux, l'invention concerne un dispositif tel que défini précédemment, dans lequel le moyen B est équipé d'un système permettant la formation de bulles, en particulier de bulles de taille contrôlée, plus particulièrement de bulles d'une taille moyenne comprise de 10µm à 50µm, à partir du CO₂ produit par la source de CO₂.

La formation de petites bulles, de l'ordre du micromètre présente l'avantage d'augmenter la surface de contact avec le moyen B et ainsi d'améliorer le transfert du CO₂.
Selon un mode de réalisation avantageux, l'invention concerne un dispositif tel que défini précédemment, dans lequel les moyens A et B sont équipés d'une sortie liquide et d'une sortie gaz, lesdites sorties étant telles que :
- les sorties liquide des moyens A et B alimentent le système de culture en première et deuxième compositions aqueuses, respectivement ;
- la sortie gaz du moyen A est reliée au moyen B ;
- la sortie gaz du moyen B est reliée au système de culture.

Ainsi, selon ce mode de réalisation, les fumées excédentaires sont avantageusement injectées dans les systèmes de culture, après traversée du moyen B.

Selon un mode de réalisation particulier, l'invention concerne un dispositif tel que défini précédemment, dans lequel ladite source de CO₂ est directement liée aux moyens A et B, et les moyens A et B sont équipés d'une sortie liquide et d'une sortie gaz, la sortie gaz du moyen A étant en particulier reliée au moyen B.

Selon un mode de réalisation particulier, l'invention concerne un dispositif tel que défini précédemment, dans lequel ladite source de CO₂ est directement liée au moyen A, et les moyens A et B sont équipés d'une sortie liquide et d'une sortie gaz, la sortie gaz du moyen A étant reliée au moyen B.

Selon un mode de réalisation avantageux, l'invention concerne un dispositif tel que défini précédemment, dans lequel la source de CO₂ est discontinue, et l'automate agit sur les différentes vannes reliant entres eux ledit système de culture, la première composition aqueuse ayant un pH supérieur à pH_{H} et la deuxième composition ayant un pH inférieur à pH_{B} de telle manière que :
- lorsque la source dé CO₂ produit du CO₂:
   ∘ du CO₂ est dirigé dans le moyen A pour obtenir une première composition aqueuse ayant un pH supérieur à pH_{H}, et dans le moyen B pour obtenir une deuxième composition aqueuse ayant un pH inférieur à pH_{B};
   ∘ (si désiré) du CO₂ est dirigé directement dans le système de culture si le pH est tel que pH_{B}< pH< pH_{H};
   ∘ quand le pH dudit milieu de culture atteint une limite haute, pH_{H}, du CO₂ produit par ladite source et éventuellement la deuxième composition aqueuse provenant du moyen B sont ajoutés audit milieu de culture de manière à faire baisser le pH, de préférence jusqu'à ce que le pH dudit milieu de culture atteigne une limite basse, pH_{B};
   ∘ quand le pH dudit milieu de culture atteint ladite limite basse, pH_{B}, la première composition aqueuse provenant du moyen A est ajoutée audit milieu de culture de manière à faire monter le pH, de préférence jusqu'à ce que le pH dudit milieu de culture atteigne une valeur intermédiaire, pH_{I} avec pH_{B}< pH_{I}< pH_{H} ;
- lorsque la source de CO₂ ne produit pas de CO₂:
   ∘ la premièré composition aqueuse provenant du moyen A est ajoutée audit milieu de culture de manière à apporter du carbone dissous dans ledit milieu de culture, provoquant une augmentation du pH jusqu'à ce que de préférence le pH dudit milieu de culture atteigne une valeur intermédiaire, pH_{I} avec pH_{B}< pH_{I}< pH_{H};
   ∘ quand le pH dudit milieu de culture atteint une limite haute, pH_{H}, la deuxième composition aqueuse provenant du moyen B est ajoutée audit milieu de culture de manière à faire baisser le pH, de préférence jusqu'à ce que le pH dudit milieu de culture atteigne une limite basse, pH_{B}.

Les deux actions décrites lorsque la source de CO₂ ne produit pas de CO₂ peuvent être inversées. En d'autres termes, il n'y a pas d'ordre défini dans le cas général. De plus, ces actions peuvent être répétées autant que nécessaire.

Selon un mode de réalisation avantageux, lorsque la source de CO₂ ne produit pas de CO₂:
∘ la première composition aqueuse provenant du moyen A est ajoutée audit milieu de culture de manière à apporter du carbone dissous dans ledit milieu de culture, provoquant une augmentation du pH jusqu'à ce que de préférence le pH dudit milieu de culture atteigne une valeur intermédiaire, pH_{I}, avec pH_{B}< pH_{I}< pH_{H}; puis
∘ quand le pH dudit milieu de culture atteint une limite haute, pH_{H}, la deuxième composition aqueuse provenant du moyen B est ajoutée audit milieu de culture de manière à faire baisser le pH, de préférence jusqu'à ce que le pH dudit milieu de culture atteigne une limite basse, pH_{B}.

En particulier, entre les deux actions mentionnées ci-dessus, le pH augmente, en particulier de pH_{I}, jusqu'à pH_{H} de part la consommation du carbone dissous dans le milieu de culture par les organismes photosynthétiques en croissance.

Selon un mode de réalisation avantageux, lorsque la source de CO₂ ne produit pas de CO₂:
∘ quand le pH dudit milieu de culture atteint une limite haute, pH_{H}, la deuxième composition aqueuse provenant du moyen B est ajoutée audit milieu de culture de manière à faire baisser le pH, de préférence jusqu'à ce que le pH dudit milieu de culture atteigne une limite basse, pH_{B}; puis
∘ la première composition aqueuse provenant du moyen A est ajoutée audit milieu de culture de manière à apporter du carbone dissous dans ledit milieu de culture, provoquant une augmentation du pH jusqu'à ce que de préférence le pH dudit milieu de culture atteigne une valeur intermédiaire, pH_{I}, avec pH_{B}< pH_{I}< pH_{H}.

En particulier, le pH augmente avant la première action mentionnée ci-dessus, jusqu'à pH_{H} du fait de la consommation du carbone dissous dans le milieu de culture par les organismes photosynthétiques en croissance. Les première et deuxième actions s'enchaînent ensuite.

### FIGURES

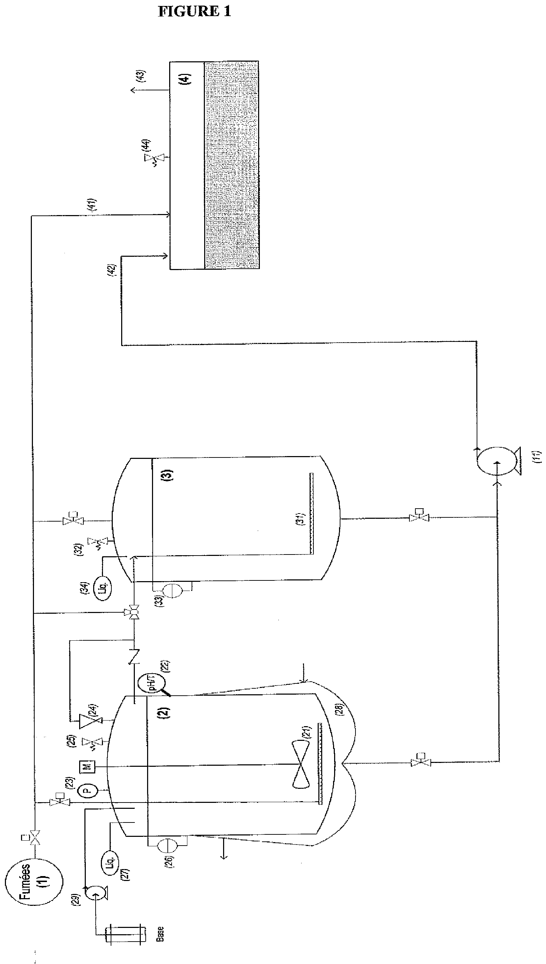

La **figure 1** représente un dispositif selon la présente invention.

La cuve de carbonatation (2) est alimentée par une fumée riche en CO₂ (1). Le transfert gaz-liquide est assuré soit par mise en contact simple entre l'ambiance gazeuse et le liquide, soit par un dispositif permettant la génération de bulles de faible taille pour augmenter le transfert (21). La pression au sein de l'enceinte est une variable permettant d'ajuster la pression partielle dans la cuve. Afin d'optimiser le fonctionnement de la cuve (2), celle-ci peut être équipée d'une sonde pH/T (22), d'un capteur de pression (23), d'un déverseur de pression (24), d'une soupape de sureté (25) et d'un capteur de niveau (26) gérant l'admission du liquide à carbonater (27). Une double enveloppe (28) peut être ajoutée pour assurer la régulation en température de la cuve de carbonatation. L'asservissement d'une pompe centrifuge (29) connectée à une solution basique permet de générer une concentration en carbone inorganiqué dissous dans la solution de carbonatation en fonction de la valeur de consigne pH et de la composition en CO₂ du gaz en entrée.

La cuve d'acidification reprend le même principe que la cuve de carbonatation, avec un transfert gaz-liquide par mise en contact simple ou améliorée par un dispositif permettant la génération de bulles de faible taille (31). La cuve peut contenir une soupape de sureté (32) et un capteur de niveau (33) gérant l'admission du liquide à acidifier (34). Elle est directement alimentée en gaz par la fumée (1) et/ou par la sortie gaz de la cuve de carbonatation (2) afin de maximiser l'utilisation de la fumée (1).

Outre les éléments nécessaires au fonctionnement classique d'un photobioréacteur, l'élément (4) dispose d'arrivées de gaz (41) et de liquide carbonaté (42), d'un évent (43) ainsi que d'une soupape de sureté (44) dans le cas d'un système clos.

Les cuves de carbonatation (2) et d'acidification (3) sont connectées au photobioréacteur (4) par :
- un réseau de gaz permettant d'alimenter indépendamment en fumée (1) les éléments (2), (3) et (4) du procédé en fonction des paramètres de contrôle.
- un réseau de liquide carbonaté permettant l'alimentation du photobioréacteur (4) en solution acide ou basique suivant les besoins du photobioréacteur (4) par l'intermédiaire d'une pompe centrifuge (11).

L'automate gère le fonctionnement des électrovannes suivant la valeur de pH relevée dans le photobioréacteur (4) et la disponibilité en fumée (1). L'automatisation de la cuve de carbonatation (2) est également assurée par l'automate.

Les cuves de carbonatation et d'acidification sont asservies via l'automate à la mesure du pH dans le système de culture. En effet, la carbonatation est privilégiée à pH basique, et la consommation biologique du carbone dans le réacteur a tendance également à basifier le milieu. Les 2 cuves/solutions liquides sont pour cela associées à un mode de régulation spécifique basé sur la mesure du pH dans le système de culture, pour à la fois fournir le carbone dissous en quantité suffisante à la croissance, et maintenir le pH optimum de croissance. Cela est basé sur la définition de 2 consignes pH (consigne haute pH_{H} et consigne basse pH_{B}) encadrant la valeur optimale de croissance (pH_{I}). Au final, on a donc pH_{B}< pH_{I}<pH_{H}.

La consommation du carbone dissous par la croissance photosynthétique provoquant une augmentation du pH dans le système de culture, quand la consigne haute pH_{H} est atteinte, la solution acide est injectée jusqu'à atteindre la consigne basse pH_{B}.

Quand la consigne basse pH_{B} est atteinte, la solution carbonatée (basique, pH>pH_{I}) est injectée jusqu'à atteindre le pH optimum de croissance.

La consommation du carbone dissous provoque une basification du milieu jusqu'à atteindre la consigne haute pH_{H}, menant à une reproduction du cycle.

A noter que les consignes pH_{B} et pH_{H} peuvent être choisies très proches du pH_{I}, permettant au final de maintenir le pH à l'optimum de croissance.

### EXEMPLE

La source de CO₂ est une fumée industrielle comprenant 9% de CO₂.

Les première et deuxième compositions aqueuses, respectivement dans les cuves de carbonatation et d'acidification, sont à une température de 25°C

Dans la cuve de carbonatation, le pH et la concentration en CID de la première composition aqueuse sont liés comme indiqué dans le tableau suivant :

| **pH** | **CID (mM)** |
|---|---|
| 7,5 | 46,7 |
| 8 | 141,2 |
| 8,5 | 444,1 |
| 9 | 1441,2 |

Ainsi, l'ajout de base, notamment de base forte, permet d'augmenter la concentration en CID de la première composition aqueuse, et donc la quantité de CID stockée dans la cuve de carbonatation.

Dans la cuve d'acidification, le pH de la deuxième composition aqueuse, à l'équilibre, est de 4,42 et la concentration en CID de 3mM.

## Revendications

1. Utilisation d'une première composition aqueuse ayant un pH supérieur à pH_{H} et d'une deuxième composition ayant un pH inférieur à pH_{B} pour la culture d'organismes photosynthétiques, choisis parmi les microalgues, les cyanobactéries et les macroalgues, dans un système de culture comprenant un milieu de culture,
dans laquelle :
- ladite première composition aqueuse ayant un pH supérieur à pH_{H} est obtenue par mise en contact de CO₂ produit par une source de CO₂, continue ou discontinue, d'une base, d'eau et éventuellement de tout ou partie des constituants d'un milieu de culture algal;
- la deuxième composition aqueuse ayant un pH inférieur à pH_{B} est obtenue par dissolution de CO₂ produit par ladite source dans de l'eau ou une solution aqueuse contenant tout ou partie des constituants d'un milieu de culture algal;
le pH dudit milieu de culture étant tel que :
- quand le pH dudit milieu de culture atteint une limite haute, pH_{H}, la deuxième composition aqueuse et/ou du CO₂ produit par ladite source sont ajoutés audit milieu de culture de manière à faire baisser le pH, de préférence jusqu'à ce que le pH dudit milieu de culture atteigne une limite basse, pH_{B};
- quand le pH dudit milieu de culture atteint ladite limite basse, pH_{B}, la première composition aqueuse est ajoutée audit milieu de culture de manière à faire monter le pH, de préférence jusqu'à ce que le pH dudit milieu de culture atteigne une valeur intermédiaire, pH_{I}, avec pH_{B}< pH_{I}< pH_{H}; et
- éventuellement, quand le pH dudit milieu de culture est inférieur à pH_{H}, en particulier à pH_{I}, la première composition aqueuse est ajoutée audit milieu de culture de manière à faire monter le pH, en particulier jusqu'à ce que le pH dudit milieu de culture atteigne la valeur intermédiaire pH_{I}.

2. Procédé de culture d'organismes photo synthétiques, choisis parmi les microalgues, les cyanobactéries et les macroalgues, à l'aide d'une source de CO₂, continue ou discontinue, dans lequel le CO₂ est dirigé au moyen de conduites et de vannes commandées de préférence par un automate:
- dans un système de culture comprenant un milieu de culture d'organismes photosynthétiques; et/ou
- dans de l'eau ou une solution aqueuse contenant tout ou partie des constituants d'un milieu de culture algal, pour obtenir en présence d'une base une première composition aqueuse ayant un pH supérieur à pH_{H}; et/ou
- dans de l'eau ou une solution aqueuse contenant tout ou partie des constituants d'un milieu de culture algal, pour obtenir une deuxième composition aqueuse ayant un pH inférieur à pH_{B}, par dissolution de CO₂ produit par ladite source dans l'eau ;
procédé **caractérisé en ce que**, lors de la culture d'organismes photosynthétiques dans ledit milieu de culture pour obtenir une biomasse d'organismes photosynthétiques à l'aide du CO₂ provenant de la dite source et/ou des ions carbonate et de l'acide carbonique respectivement contents dans les première et deuxième compositions aqueuses:
i. quand le pH dudit milieu de culture atteint une limite haute, pH_{H}, la deuxième composition aqueuse et/ou du CO₂ produit par ladite source sont ajoutés audit milieu de culture de manière à faire baisser le pH, de préférence jusqu'à ce que le pH dudit milieu de culture atteigne une limite basse, pH_{B};
ii. quand le pH dudit milieu de culture atteint ladite limite basse, pH_{B}, la première composition aqueuse est ajoutée audit milieu de culture de manière à faire monter le pH, de préférence jusqu'à ce que le pH dudit milieu de culture atteigne une valeur intermédiaire, pH_{I}, avec pH_{B}< pH_{I}< pH_{H}; et
iii. éventuellement, quand le pH dudit milieu de culture est inférieur à pH_{H}, en particulier à pH_{I}, la première composition aqueuse est ajoutée audit milieu de culture de manière à faire monter le pH, en particulier jusqu'à ce que le pH dudit milieu de culture atteigne la valeur intermédiaire pH_{I};
ladite valeur de pH_{I}, étant notamment comprise de 6 à 10 dans le milieu de culture ;
ledit pH_{H} étant notamment tel que pH_{H} = pH_{I} + x dans le milieu de culture, x étant compris de 0,02 à 1,5, en particulier de 0,1 à 0,2 ;
ledit pH_{B} étant notamment tel que pH_{B} = pH_{I} - y dans le milieu de culture, y étant compris de 0,02 à 1,5, en particulier de 0,1 à 0,2 ;
ladite base étant notamment choisie dans le groupe constitué de l'hydroxyde de sodium et de l'hydroxyde dè potassium ;
ledit milieu de culture étant notamment à une température comprise de 15°C à 35°C.

3. Procédé selon la revendication 2, dans lequel les organismes photosynthétiques sont choisis dans le groupe constitué :
- des microalgues, en particulier des microalgues des genres *Chlorella, Nannochloropsis, Chlamydomonas, Tetraselmis, Scendesmus, Parachlorella, Porphyridium, Botryococcus* et *Neochloris;*
- des cyanobactéries, en particulier des genres *Arthrospira, Aphazomenon* et *Synechocystis;* et
- des macroalgues, en particulier les macroalgues Ulva, Fucus, Palmaria.

4. Procédé selon la revendication 2, dans lequel la source de CO₂ est constituée de fumées industrielles, ladite la source de CO₂ étant en particulier choisie dans le groupe constitué des émissions de chaudière, de centrale thermique, de cimenterie, de sidérurgie, de raffinerie, d'usine de fabrication d'ammoniac, des procédés de fermentation et des procédés de digestion anaérobie.

5. Procédé selon la revendication 2, dans lequel la source de CO₂ est discontinue, et l'automate agit sur les différentes vannes reliant entres eux un système de culture d'organismes photo synthétiques comprenant ledit milieu de culture, la première composition aqueuse ayant un pH supérieur à pH_{H} et la deuxième composition ayant un pH inférieur à pH_{B} de telle manière que :
- lorsque la source de CO₂ produit du CO₂ :
∘ du CO₂ est dirigé dans de l'eau ou une solution aqueuse contenant tout ou partie des constituants d'un milieu de culture algal, pour obtenir en présence d'une base ladite première composition aqueuse, et dans de l'eau ou une solution aqueuse contenant tout ou partie des constituants d'un milieu de culture algal pour obtenir ladite deuxième composition aqueuse par dissolution de CO₂ produit par ladite source dans l'eau ou la solution aqueuse ;
∘ (si désiré) du CO₂ est dirigé directement dans le système de culture si le pH est tel que pH_{B}< pH< pH_{H};
∘ quand le pH dudit milieu de culture atteint une limite haute, pH_{H}, du CO₂ produit par ladite source et éventuellement la deuxième composition aqueuse sont ajoutés audit milieu de culture de manière à faire baisser le pH, de préférence jusqu'à ce que le pH dudit milieu de culture atteigne une limite basse, pH_{B};
∘ quand le pH dudit milieu de culture atteint ladite limite basse, pH_{B}, le CO₂ produit par ladite source n'est plus ajouté audit milieu de culture, et si désiré, la première composition aqueuse est ajoutée audit milieu de culture de manière à faire monter le pH, de préférence jusqu'à ce que le pH dudit milieu de culture atteigne une valeur intermédiaire, pH_{I}, avec pH_{B}< pH_{I}< pH_{H};
- lorsque la source de CO₂ ne produit pas de CO₂:
∘ la première composition aqueuse est ajoutée au milieu de culture de manière à apporter du carbone dissous dans ledit milieu de culture, provoquant une augmentation du pH jusqu'à ce que de préférence le pH dudit milieu de culture atteigne une valeur intermédiaire, pH_{I}, avec pH_{B}< pH_{I}< pH_{H};
∘ quand le pH dudit milieu de culture atteint une limite haute, pH_{H}, la deuxième composition aqueuse est ajoutée audit milieu de culture de manière à faire baisser le pH, de préférence jusqu'à ce que le pH dudit milieu de culture atteigne une limite basse, pH_{B}.

6. Procédé selon la revendication 2, dans lequel le système de culture est un système fermé.

7. Procédé selon la revendication 2, dans lequel le système de culture est un système ouvert.

8. Dispositif pour la culture d'organismes photosynthétiques, choisis parmi les microalgues, les cyanobactéries et les macroalgues, à l'aide d'une source de CO₂, continue ou discontinue, comprenant les éléments suivants:
- un moyen A de captation du CO₂ produit par ladite source, moyen comprenant de l'eau ou une solution aqueuse contenant tout ou partie des constituants d'un milieu de culture algal, et une base, et permettant l'obtention d'une première composition aqueuse ayant un pH supérieur à pH_{H};
- un moyen B de captation du CO₂ produit par ladite source, moyen comprenant de l'eau ou une solution aqueuse contenant tout ou partie des constituants d'un milieu de culture algal, et permettant l'obtention d'une deuxième composition aqueuse ayant un pH inférieur à pH_{B};
- un système de culture desdits organismes photosynthétiques, équipé de moyens de mesure du pH;
- des conduites et des vannes reliant entre eux la source de CO₂, le système de culture desdits organismes photosynthétiques et les moyens A et B
- un système de commande et de contrôle des flux gazeux et liquides entre la source de CO₂, le système de culture desdits organismes photosynthétiques et les moyens A et B, de préférence un automate, **caractérisé en ce que** le système de commande est agencé de façon à ce que :
∘ le CO₂ produit par ladite source est dirigé vers ledit système de culture, et/ou lesdits moyens A et B de captation du CO₂;
∘ quand le pH dudit milieu de culture atteint une limite haute, pH_{H}, la deuxième composition aqueuse provenant du moyen B et/ou du CO₂ produit par ladite source sont ajoutés audit milieu de culture de manière à faire baisser le pH, de préférence jusqu'à ce que le pH dudit milieu de culture atteigne une limite basse, pH_{B};
∘ quand le pH dudit milieu de culture atteint ladite limite basse, pH_{B}, la première composition aqueuse provenant du moyen A est ajoutée audit milieu de culture de manière à faire monter le pH, de préférence jusqu'à ce que le pH dudit milieu de culture atteigne une valeur intermédiaire, pH_{I}, avec pH_{B}< pH_{I}< pH_{H} ; et
∘ ventuellement, quand le pH dudit milieu de culture est inférieur à pH_{H}, en particulier à pH_{I}, la première composition aqueuse est ajoutée audit milieu de culture de manière à faire monter le pH, en particulier jusqu'à ce que le pH dudit milieu de culture atteigne la valeur intermédiaire pH_{I}.
ledit moyen A étant notamment équipé d'une sonde de pH et/ou de température ; et/ou ledit moyen A étant notamment équipé d'un capteur de pression et d'un déverseur de pression ; et/ou
ledit moyen A étant notamment équipé d'un système permettant la formation de bulles, en particulier de bulles de taille contrôlée, plus particulièrement de bulles d'une taille moyenne comprise de 10µm à 50µm, à partir du CO₂ produit par la source de CO₂;
ledit moyen B étant notamment équipé d'un système permettant la formation de bulles, en particulier de bulles de taille contrôlée, plus particulièrement de bulles d'une taille moyenne comprise de 10µm à 50µm, à partir du CO₂ produit par la source de CO₂.

9. Dispositif selon la revendication 8, dans lequel le système de culture est un système fermé.

10. Dispositif selon la revendication 8, dans lequel le système de culture est un système ouvert.

11. Dispositif selon la revendication 8, dans lequel les moyens A et B sont équipés d'une sortie liquide et d'une sortie gaz, lesdites sorties étant telles que :
- les sorties liquide des moyens A et B alimentent le système de culture en première et deuxième compositions aqueuses, respectivement ;
- la sortie gaz du moyen A est reliée au moyen B ;
- la sortie gaz du moyen B est reliée au système de culture.

12. Dispositif selon la revendication 8, dans lequel la source de CO₂ est discontinue, et l'automate agit sur les différentes vannes reliant entres eux ledit système de culture, la première composition aqueuse ayant un pH supérieur à pH_{H} et la deuxième composition ayant un pH inférieur à pH_{B} de telle manière que :
- lorsque la source de CO₂ produit du CO₂ :
∘ du CO₂ est dirigé dans le moyen A pour obtenir une première composition aqueuse ayant un pH supérieur à pH_{H}, et dans le moyen B pour obtenir une deuxième composition aqueuse ayant un pH inférieur à pH_{B};
∘ (si désiré) du CO₂ est dirigé directement dans le système de culture si le pH est tel que pH_{B}< pH< pH_{H};
∘ quand le pH dudit milieu de culture atteint une limite haute, pH_{H}, du CO₂ produit par ladite source et éventuellement la deuxième composition aqueuse provenant du moyen B sont ajoutés audit milieu de culture de manière à faire baisser le pH, de préférence jusqu'à ce que le pH dudit milieu de culture atteigne une limite basse, pH_{B};
∘ quand le pH dudit milieu de culture atteint ladite limite basse, pH_{B}, la première composition aqueuse provenant du moyen A est ajoutée audit milieu de culture de manière à faire monter le pH, de préférence jusqu'à ce que le pH dudit milieu de culture atteigne une valeur intermédiaire, pH_{I}, avec pH_{B}< pH_{I}< pH_{H};
- lorsque la source de CO₂ ne produit pas de CO₂:
∘ la première composition aqueuse provenant du moyen A est ajoutée audit milieu de culture de manière à apporter du carbone dissous dans ledit milieu de culture, provoquant une augmentation du pH jusqu'à ce que de préférence le pH dudit milieu de culture atteigne une valeur intermédiaire, pH_{I}, avec pH_{B}< pH_{I}< pH_{H}.
quand le pH dudit milieu de culture atteint une limite haute, pH_{H}, la deuxième composition aqueuse provenant du moyen B est ajoutée audit milieu de culture de manière à faire baisser le pH, de préférence jusqu'à ce que le pH dudit milieu de culture atteigne une limite basse, pH_{B}.

## Patentansprüche

1. Verwendung einer ersten wässrigen Zusammensetzung mit einem pH größer als pH_{H} und einer zweiten Zusammensetzung mit einem pH kleiner als pH_{B} zur Anzucht von fotosynthetischen Organismen, die aus Mikroalgen, Cyanobakterien und Makroalgen ausgewählt werden, in einem Anzuchtsystem, das ein Anzuchtmedium umfasst,
wobei:
- die erste wässrige Zusammensetzung mit einem pH größer als pH_{H} durch Inberührungbringen von CO₂, das von einer kontinuierlichen oder diskontinuierlichen CO₂-Quelle erzeugt wird, von einer Base, von Wasser und gegebenenfalls von allen oder einem Teil der Bestandteile eines Algenanzuchtmediums erhalten wird;
- die zweite wässrige Zusammensetzung mit einem pH kleiner als pH_{B} durch Lösen von CO₂, das von der Quelle erzeugt wird, in Wasser oder einer wässrigen Lösung, enthaltend alle oder einen Teil der Bestandteile eines Algenanzuchtmediums, erhalten wird;
wobei der pH des Anzuchtmediums derart ist, dass:
- wenn der pH des Anzuchtmediums eine Obergrenze, pH_{H}, erreicht, die zweite wässrige Zusammensetzung und/oder CO₂, das von der Quelle erzeugt wird, dem Anzuchtmedium derart zugesetzt werden, dass der pH gesenkt wird, vorzugsweise bis der pH des Anzuchtmediums eine Untergrenze, pH_{B}, erreicht;
- wenn der pH des Anzuchtmediums die Untergrenze, pH_{B}, erreicht, die erste wässrige Zusammensetzung dem Anzuchtmedium derart zugesetzt wird, dass der pH erhöht wird, vorzugsweise bis der pH des Anzuchtmediums einen Zwischenwert, pH_{I}, erreicht, wobei pH_{B} < pH_{I}< pH_{H}; und
- wenn gegebenenfalls der pH des Anzuchtmediums kleiner ist als pH_{H}, insbesondere als pH_{I,} die erste wässrige Zusammensetzung dem Anzuchtmedium derart zugesetzt wird, dass der pH erhöht wird, insbesondere bis der pH des Anzuchtmediums den Zwischenwert pH_{I} erreicht.

2. Verfahren zur Anzucht von fotosynthetischen Organismen, die aus Mikroalgen, Cyanobakterien und Makroalgen ausgewählt werden, mit Hilfe einer kontinuierlichen oder diskontinuierlichen CO₂-Quelle, wobei das CO₂ mittels Leitungen und Ventilen geleitet wird, die vorzugsweise von einem Automaten gesteuert werden:
- in einem Anzuchtsystem, das ein Anzuchtmedium von fotosynthetischen Organismen umfasst; und/oder
- in Wasser oder einer wässrigen Lösung, enthaltend alle oder einen Teil der Bestandteile eines Algenanzuchtmediums, um in Anwesenheit einer Base eine erste wässrige Zusammensetzung mit einem pH größer als pH_{H} zu erhalten; und/oder
- in Wasser oder einer wässrigen Lösung, enthaltend alle oder einen Teil der Bestandteile eines Algenanzuchtmediums, um eine zweite wässrige Zusammensetzung mit einem pH kleiner als pH_{H} zu erhalten, durch Lösen des CO₂, das von der Quelle erzeugt wird, in Wasser;
wobei das Verfahren **dadurch gekennzeichnet ist, dass** bei der Anzucht der fotosynthetischen Organismen in dem Anzuchtmedium, um eine Biomasse von fotosynthetischen Organismen mit Hilfe des CO₂, das von der Quelle stammt, und/oder von Carbonationen und von Carbonsäure zu erhalten, die jeweils in der ersten und zweiten wässrigen Zusammensetzung enthalten sind:
i. wenn der pH des Anzuchtmediums eine Obergrenze, pH_{H}, erreicht, die zweite wässrige Zusammensetzung und/oder CO₂, das von der Quelle erzeugt wird, dem Anzuchtmedium derart zugesetzt werden, dass der pH gesenkt wird, vorzugsweise bis der pH des Anzuchtmediums eine Untergrenze, pH_{B}, erreicht;
ii. wenn der pH des Anzuchtmediums die Untergrenze, pH_{B}, erreicht, die erste wässrige Zusammensetzung dem Anzuchtmedium derart zugesetzt wird, dass der pH erhöht wird, vorzugsweise bis der pH des Anzuchtmediums einen Zwischenwert, pH_{I}, erreicht, wobei pH_{B} < pH, < pH_{H}; und
iii. wenn gegebenenfalls der pH des Anzuchtmediums kleiner ist als pH_{H}, insbesondere als pH_{I} die erste wässrige Zusammensetzung dem Anzuchtmedium derart zugesetzt wird, dass der pH erhöht wird, insbesondere bis der pH des Anzuchtmediums den Zwischenwert pH_{I} erreicht;
wobei der Wert pH_{I} insbesondere von 6 bis 10 in dem Anzuchtmedium beträgt;
wobei pH_{H} insbesondere derart ist, dass pH_{H} = pH_{I} + x in dem Anzuchtmedium, wobei x von 0,02 bis 1,5, vorzugsweise von 0,1 bis 0,2 beträgt;
wobei pH_{B} insbesondere derart ist, dass pH_{B} = pH_{I} - y in dem Anzuchtmedium, wobei y von 0,02 bis 1,5, vorzugsweise von 0,1 bis 0,2 beträgt;
wobei die Base insbesondere ausgewählt ist aus der Gruppe bestehend aus Natriumhydroxid und Kaliumhydroxid;
wobei das Anzuchtmedium insbesondere eine Temperatur von 15 °C bis 35 °C aufweist.

3. Verfahren nach Anspruch 2, wobei die fotosynthetischen Organismen ausgewählt werden aus der Gruppe bestehend aus:
- Mikroalgen, insbesondere Mikroalgen der Gattungen *Chlorella, Nannochloropsis, Chlamydomonas, Tetraselmis, Scendesmus, Parachlorella, Porphyridium, Botryococcus* und *Neochloris;*
- Cyanobakterien, insbesondere der Gattungen *Arthrospira, Aphazomenon* und *Synechocystis;* und
- Makroalgen, insbesondere den Makroalgen Ulva, Fucus, Palmaria.

4. Verfahren nach Anspruch 2, wobei die CO₂-Quelle aus Industrieabgasen besteht, wobei die CO₂-Quelle insbesondere ausgewählt wird aus der Gruppe bestehend aus Emissionen von Heizkesseln, Kraftwerken, Zementfabriken, Eisen- und Stahlwerken, Raffinerien, Anlagen zur Herstellung von Ammoniak, Fermentationsprozessen und Prozessen zur anaeroben Vergärung.

5. Verfahren nach Anspruch 2, wobei die CO₂-Quelle diskontinuierlich ist, und der Automat auf verschiedene Ventile derart einwirkt, die ein System zur Anzucht von fotosynthetischen Organismen miteinander verbinden, welches das Anzuchtmedium umfasst, wobei die erste wässrige Zusammensetzung einen pH größer als pH_{H} aufweist, und die zweite Zusammensetzung einen pH kleiner als pH_{B} aufweist, dass:
- wenn die CO₂-Quelle CO₂ erzeugt:
∘ CO₂ geleitet wird in das Wasser oder eine wässrige Lösung, enthaltend alle oder einen Teil der Bestandteile eines Algenanzuchtmediums, um in Anwesenheit einer Base die erste wässrige Zusammensetzung zu erhalten, oder in das Wasser oder eine wässrige Lösung, enthaltend alle oder einen Teil der Bestandteile eines Algenanzuchtmediums, um die zweite wässrige Zusammensetzung durch Lösen des CO₂, das von der Quelle erzeugt wird, in dem Wasser oder der wässrigen Lösung zu erhalten;
∘ (wenn gewünscht) CO₂ direkt in das Anzuchtsystem geleitet wird, wenn der pH derart ist, dass pH_{B} < pH_{I} < pH_{H};
∘ wenn der pH des Anzuchtmediums eine Obergrenze, pH_{H}, erreicht, CO₂, das von der Quelle erzeugt wird, und gegebenenfalls die zweite wässrige Zusammensetzung dem Anzuchtmedium derart zugesetzt werden, dass der pH gesenkt wird, vorzugsweise bis der pH des Anzuchtmediums eine Untergrenze, pH_{B}, erreicht;
∘ wenn der pH des Anzuchtmediums die Untergrenze, pH_{B}, erreicht, das CO₂, das von der Quelle erzeugt wird, nicht mehr dem Anzuchtmedium zugesetzt wird, und, wenn gewünscht, die erste wässrige Zusammensetzung dem Anzuchtmedium derart zugesetzt wird, dass der pH erhöht wird, vorzugsweise bis der pH des Anzuchtmediums einen Zwischenwert, pH_{I}, erreicht, wobei pH_{B} < pH_{I}< pH_{H};
- wenn die CO₂-Quelle kein CO₂ erzeugt:
∘ die erste wässrige Zusammensetzung dem Anzuchtmedium derart zugesetzt wird, dass gelöster Kohlenstoff in das Anzuchtmedium eingebracht wird, wodurch eine Zunahme des pH hervorgerufen wird, vorzugsweise bis der pH des Anzuchtmediums einen Zwischenwert, pH_{I} erreicht, wobei pH_{B} < pH_{I} < pH_{H};
∘ wenn der pH des Anzuchtmediums eine Obergrenze, pH_{H}, erreicht, die zweite wässrige Zusammensetzung dem Anzuchtmedium derart zugesetzt wird, dass der pH gesenkt wird, vorzugsweise bis der pH des Anzuchtmediums eine Untergrenze, pH_{B}, erreicht.

6. Verfahren nach Anspruch 2, wobei das Anzuchtsystem ein geschlossenes System ist.

7. Verfahren nach Anspruch 2, wobei das Anzuchtsystem ein offenes System ist.

8. Vorrichtung zur Anzucht von fotosynthetischen Organismen, die aus Mikroalgen, Cyanobakterien und Makroalgen ausgewählt werden, mit Hilfe einer kontinuierlichen oder diskontinuierlichen CO₂-Quelle, umfassend die folgenden Elemente:
- ein Mittel A zum Auffangen von CO₂, das von der Quelle erzeugt wird, wobei das Mittel Wasser oder eine wässrige Lösung, enthaltend alle oder einen Teil der Bestandteile eines Algenanzuchtmediums, und eine Base umfasst, und das Erhalten einer ersten wässrigen Zusammensetzung mit einem pH größer pH_{H} gestattet;
- ein Mittel B zum Auffangen von CO₂, das von der Quelle erzeugt wird, wobei das Mittel Wasser oder eine wässrige Lösung, enthaltend alle oder einen Teil der Bestandteile eines Algenanzuchtmediums, umfasst, und das Erhalten einer zweiten wässrigen Zusammensetzung mit einem pH kleiner pH_{B} gestattet;
- ein System zur Anzucht der fotosynthetischen Organismen, das mit Mitteln zur Messung des pH ausgestattet ist;
- Leitungen und Ventile, welche die CO₂-Quelle, das System zur Anzucht der fotosynthetischen Organismen und die Mittel A und B miteinander verbinden;
- ein System zur Regelung und zur Steuerung von gasförmigen und flüssigen Strömen zwischen der CO₂-Quelle, dem System zur Anzucht der fotosynthetischen Organismen und den Mitteln A und B, vorzugsweise einen Automaten, **dadurch gekennzeichnet, dass** das Regelungssystem derart eingerichtet ist, dass:
∘ das CO₂, das von der Quelle erzeugt wird, zu dem Anzuchtsystem und/oder den Mitteln A und B zum Auffangen von CO₂ geleitet wird;
∘ wenn der pH des Anzuchtmediums eine Obergrenze, pH_{H}, erreicht, die zweite wässrige Zusammensetzung, die von dem Mittel B stammt, und/oder CO₂, das von der Quelle erzeugt wird, dem Anzuchtmedium derart zugesetzt werden, dass der pH gesenkt wird, vorzugsweise bis der pH des Anzuchtmediums eine Untergrenze, pH_{B}, erreicht;
∘ wenn der pH des Anzuchtmediums die Untergrenze, pH_{B}, erreicht, die erste wässrige Zusammensetzung, die von dem Mittel A stammt, dem Anzuchtmedium derart zugesetzt wird, dass der pH erhöht wird, vorzugsweise bis der pH des Anzuchtmediums einen Zwischenwert, pH_{I}, erreicht, wobei pH_{B} < pH_{I} < pH_{H}; und
∘ wenn gegebenenfalls der pH des Anzuchtmediums kleiner ist als pH_{H}, insbesondere als pH_{I}, die erste wässrige Zusammensetzung dem Anzuchtmedium derart zugesetzt wird, dass der pH erhöht wird, insbesondere bis der pH des Anzuchtmediums den Zwischenwert pH_{I} erreicht;
wobei das Mittel A insbesondere mit einer Sonde für den pH und/oder die Temperatur ausgestattet ist;
und/oder
wobei das Mittel A insbesondere mit einem Drucksensor und einem Druckentlaster ausgestattet ist; und/oder
wobei das Mittel A insbesondere mit einem System ausgestattet ist, das die Bildung von Bläschen, insbesondere von Bläschen mit einer gesteuerten Größe, bevorzugter von Bläschen mit einer mittleren Größe von 10 µm bis 50 µm, aus dem CO₂, das von der CO₂-Quelle erzeugt wird, gestattet;
wobei das Mittel B insbesondere mit einem System ausgestattet ist, das die Bildung von Bläschen, insbesondere von Bläschen mit einer gesteuerten Größe, bevorzugter von Bläschen mit einer mittleren Größe von 10 µm bis 50 µm, aus dem CO₂, das von der CO₂-Quelle erzeugt wird, gestattet.

9. Vorrichtung nach Anspruch 8, wobei das Anzuchtsystem ein geschlossenes System ist.

10. Vorrichtung nach Anspruch 8, wobei das Anzuchtsystem ein offenes System ist.

11. Vorrichtung nach Anspruch 8, wobei die Mittel A und B mit einem Flüssigausgang und mit einem Gasausgang ausgestattet sind, wobei die Ausgänge derart sind, dass:
- die Flüssigausgänge der Mittel A und B das Anzuchtsystem jeweils mit einer ersten und zweiten wässrigen Zusammensetzung versorgen;
- der Gasausgang des Mittels A mit dem Mittel B verbunden ist;
- der Gasausgang des Mittels B mit dem Anzuchtsystem verbunden ist.

12. Vorrichtung nach Anspruch 8, wobei die CO₂-Quelle diskontinuierlich ist, und der Automat auf die verschiedenen Ventile derart einwirkt, die das Anzuchtsystem miteinander verbinden, wobei die erste wässrige Zusammensetzung einen pH größer als pH_{H} aufweist, und die zweite Zusammensetzung einen pH kleiner als pH_{B} aufweist, dass:
- wenn die CO₂-Quelle CO₂ erzeugt:
∘ CO₂ geleitet wird in das Mittel A, um eine erste wässrige Zusammensetzung mit einem pH größer als pH_{H} zu erhalten, und in das Mittel B, um eine zweite wässrige Zusammensetzung mit einem pH kleiner als pH_{B} zu erhalten;
∘ (wenn gewünscht) CO₂ direkt in das Anzuchtsystem geleitet wird, wenn der pH derart ist, dass pH_{B} < pH_{I} < pH_{H};
∘ wenn der pH des Anzuchtmediums eine Obergrenze, pH_{H}, erreicht, CO₂, das von der Quelle erzeugt wird, und gegebenenfalls die zweite wässrige Zusammensetzung, die von dem Mittel B stammt, dem Anzuchtmedium derart zugesetzt werden, dass der pH gesenkt wird, vorzugsweise bis der pH des Anzuchtmediums eine Untergrenze, pH_{B}, erreicht;
∘ wenn der pH des Anzuchtmediums die Untergrenze, pH_{B}, erreicht, die erste wässrige Zusammensetzung, die von dem Mittel A stammt, dem Anzuchtmedium derart zugesetzt wird, dass der pH erhöht wird, vorzugsweise bis der pH des Anzuchtmediums einen Zwischenwert, pH_{I}, erreicht, wobei pH_{B} < pH_{I} < pH_{H};
- wenn die CO₂-Quelle kein CO₂ erzeugt:
∘ die erste wässrige Zusammensetzung, die von dem Mittel A stammt, dem Anzuchtmedium derart zugesetzt wird, dass gelöster Kohlenstoff in das Anzuchtmedium eingebracht wird, wodurch eine Zunahme des pH hervorgerufen wird, vorzugsweise bis der pH des Anzuchtmediums einen Zwischenwert, pH_{I}, erreicht, wobei pH_{B} < pH_{I} < pH_{H};
∘ wenn der pH des Anzuchtmediums eine Obergrenze, pH_{H}, erreicht, die zweite wässrige Zusammensetzung, die von dem Mittel B stammt, dem Anzuchtmedium derart zugesetzt wird, dass der pH gesenkt wird, vorzugsweise bis der pH des Anzuchtmediums eine Untergrenze, pH_{B}, erreicht.

## Claims

1. Use of a first aqueous composition having a pH greater than pH_{H} and of a second composition having a pH lower than pH_{B} for the culture of photosynthetic organisms, selected from microalgae, cyanobacteria and macroalgae, in a culture system comprising a culture medium,
in which:
- said first aqueous composition having a pH greater than pH_{H} is obtained by contacting CO₂ produced by a continuous or discontinuous CO₂ source, a base, water and possibly all or part of the constituents of an algal culture medium;
- the second aqueous composition having a pH lower than pH_{B} is obtained by dissolving CO₂ produced by said source in water or an aqueous solution containing all or part of the constituents of an algal culture medium;
the pH of said culture medium being such that:
- when the pH of said culture medium reaches an upper limit, pH_{H}, the second aqueous composition and/or CO₂ produced by said source are added to said culture medium so as to lower the pH, preferably until the pH of said culture medium reaches a lower limit, pH_{B};
- when the pH of said culture medium reaches said lower limit, pH_{B}, the first aqueous composition is added to said culture medium so as to increase the pH, preferably until the pH of said culture medium reaches an intermediate value, pH_{I}, with pH_{B}< pH_{I}< pH_{H}; and
- optionally, when the pH of said culture medium is lower than pH_{H}, in particular lower than pH_{I}, the first aqueous composition is added to said culture medium so as to increase the pH, in particular until the pH of said culture medium reaches the intermediate value pH_{I}.

2. Process for the culture of photosynthetic organisms, selected from microalgae, cyanobacteria and macroalgae, using a continuous or discontinuous CO₂ source, in which the CO₂ is directed by means of pipes and valves controlled preferably by an automaton:
- in a culture system comprising a medium for the culture of photosynthetic organisms; and/or
- in water or an aqueous solution containing all or part of the constituents of an algal culture medium so as to obtain, in the presence of a base, a first aqueous composition having a pH greater than pH_{H}; and/or
- in water or an aqueous solution containing all or part of the constituents of an algal culture medium so as to obtain a second aqueous composition having a pH lower than pH_{B}, by dissolving CO₂ produced by said source in water;
said method being **characterised in that**, during the culture of photosynthetic organisms in said culture medium, in order to obtain a biomass of photosynthetic organisms using the CO₂ originating from said source and/or the carbonate ions and the carbonic acid respectively contained in the first and second aqueous compositions:
i. when the pH of said culture medium reaches an upper limit, pH_{H}, the second aqueous composition and/or the CO₂ produced by said source are added to said culture medium in order to lower the pH, preferably until the pH of said culture medium reaches a lower limit, pH_{B};
ii. when the pH of said culture medium reaches said lower limit, pH_{B}, the first aqueous composition is added to said culture medium so as to increase the pH, preferably until the pH of said culture medium reaches an intermediate value, pH_{I}, with pH_{B}< pH_{I}< pH_{H}; and
iii. possibly, when the pH of said culture medium is lower than pH_{H}, in particular lower than pH_{I}, the first aqueous composition is added to said culture medium so as to increase the pH, in particular until the pH of said culture medium reaches the intermediate value pH_{I};
said value of pH_{I} being in particular between 6 and 10 in the culture medium;
said pH_{H} being in particular such that pH_{H} = pH_{I} + x in the culture medium, x being between 0.02 and 1.5, in particular between 0.1 and 0.2;
said pH_{B} being in particular such that pH_{B} = pH_{I} - y in the culture medium, y being between 0.02 and 1.5, in particular between 0.1 and 0.2;
said base being in particular selected from the group composed of sodium hydroxide and potassium hydroxide;
said culture medium being in particular at a temperature between 15°C and 35°C.

3. Process according to claim 2, wherein the photosynthetic organisms are selected from the group composed of:
- microalgae, in particular microalgae of the genera *Chlorella, Nannochloropsis, Chlamydomonas, Tetraselmis, Scendesmus, Parachlorella, Porphyridium, Botryococcus* and *Neochloris;*
- cyanobacteria, in particular of the genera *Arthrospira, Aphazomenon* and *Synechocystis;* and
- macroalgae, in particular the macroalgae Ulva, Fucus, Palmaria.

4. Process according to claim 2, wherein the CO₂ source is formed by industrial fumes gases, said CO₂ source being selected in particular from the group formed by emissions from boiler, thermal power plant, cement plant, steel industry, refinery, factory plant ammonia, fermentation process, and anaerobic digestion process.

5. Process according to claim 2, wherein the CO₂ source is discontinuous and the automaton acts on the various valves connecting a system for the culture of photosynthetic organisms comprising said culture medium, the first aqueous composition having a pH greater than pH_{H} and the second composition having a pH lower than pH_{B}, such that:
- when the CO₂ source produces CO₂:
∘ CO₂ is directed into water or an aqueous solution containing all or part of the constituents of an algal culture medium so as to obtain, in the presence of a base, said first aqueous composition, and is directed into water or an aqueous solution containing all or part of the constituents of an algal culture medium so as to obtain said second aqueous composition by dissolving CO₂ produced by said source in the water or the aqueous solution;
∘ (if desired) CO₂ is directed directly into the culture system if the pH is such that pH_{B}< pH< pH_{H};
∘ when the pH of said culture medium reaches an upper limit, pH_{H}, CO₂ produced by said source and possibly the second aqueous composition are added to said culture medium so as to lower the pH, preferably until the pH of said culture medium reaches a lower limit, pH_{B};
∘ when the pH of said culture medium reaches said lower limit, pH_{B}, the CO₂ produced by said source is no longer added to said culture medium, and, if desired, the first aqueous composition is added to said culture medium so as to raise the pH, preferably until the pH of said culture medium reaches an intermediate value, pH_{I}, with pH_{B}< pH_{I}< pH_{H};
- when the CO₂ source does not produce CO₂:
∘ the first aqueous composition is added to the culture medium so as to supply dissolved carbon to said culture medium, resulting in an increase of the pH until preferably the pH of said culture medium reaches an intermediate value, pH_{I}, with pH_{B}< pH_{I}< pH_{H};
∘ when the pH of said culture medium reaches an upper limit, pH_{H}, the second aqueous composition is added to said culture medium so as to lower the pH, preferably until the pH of said culture medium reaches a lower limit, pH_{B}.

6. Process according to claim 2, wherein the culture system is a closed system.

7. Process according to claim 2, wherein the culture system is an open system.

8. Device for the culture of photosynthetic organisms, selected from microalgae, cyanobacteria and macroalgae, using a continuous or discontinuous CO₂ source, said device comprising the following elements:
- a means A for capturing CO₂ produced by said source, said means comprising water or an aqueous solution containing all or part of the constituents of an algal culture medium, and a base, and making it possible to obtain a first aqueous composition having a pH greater than pH_{H};
- a means B for capturing CO₂ produced by said source, said means comprising water or an aqueous solution containing all or part of the constituents of an algal culture medium and making it possible to obtain a second aqueous composition having a pH lower than pH_{B};
- a system for the culture of said photosynthetic organisms, equipped with means for measuring pH;
- pipes and valves connecting the CO₂ source, the system for the culture of said photosynthetic organisms, and the means A and B;
- a system for controlling and regulating gaseous and liquid flows between the CO₂ source, the system for the culture of said photosynthetic organisms, and the means A and B, preferably an automaton, **characterised in that** the control system is designed such that:
∘ the CO₂ produced by said source is directed towards said culture system and/or said means A and B for capturing CO₂;
∘ when the pH of said culture medium reaches an upper limit, pH_{H}, the second aqueous composition originating from the means B and/or CO₂ produced by said source are added to said culture medium so as to lower the pH, preferably until the pH of said culture medium reaches a lower limit, pH_{B};
∘ when the pH of said culture medium reaches said lower limit, pH_{B}, the first aqueous composition originating from the means A is added to said culture medium so as to increase the pH, preferably until the pH of said culture medium reaches an intermediate value, pH_{I}, with pH_{B}< pH_{I}< pH_{H}; and
∘ possibly, when the pH of said culture medium is lower than pH_{H}, in particular lower than pH_{I}, the first aqueous composition is added to said culture medium so as to increase the pH, in particular until the pH of said culture medium reaches the intermediate value pH_{I},
said means A being in particular equipped with a pH and/or temperature probe; and/or
said means A being in particular equipped with a pressure sensor and a back-pressure regulator; and/or
said means A being in particular equipped with a system making it possible to form bubbles, in particular bubbles of controlled size, more particularly bubbles with an average size between 10µm and 50µm, from the CO₂ produced by the CO₂ source;
said means B being equipped with a system allowing the formation of bubbles, in particular bubbles of controlled size, more particularly bubbles of an average size between 10µm and 50µm, from the CO₂ produced by the CO₂ source

9. Device according to claim 8, wherein the culture system is a closed system.

10. Device according to claim 8, wherein the culture system is an open system.

11. Device according to claim 8, wherein the means A and B are equipped with a liquid outlet and a gas outlet, said outlets being such that:
- the liquid outlets of the means A and B supply the culture system with first and second aqueous compositions, respectively;
- the gas outlet of the means A is connected to the means B;
- the gas outlet of the means B is connected to the culture system.

12. Device according to claim 8, wherein the CO₂ source is discontinuous, and the automaton acts on the various valves connecting said culture system, the first aqueous composition having a pH greater than pH_{H} and the second composition having a pH lower than pH_{B}, such that:
- when the CO₂ source produces CO₂:
∘ CO₂ is directed into the means A so as to obtain a first aqueous composition having a pH greater than pH_{H}, and into the means B so as to obtain a second aqueous composition having a pH lower than pH_{B};
∘ (if desired) CO₂ is directed directly into the culture system if the pH is such that pH_{B}< pH< pH_{H};
∘ when the pH of said culture medium reaches an upper limit, pH_{H}, CO₂ produced by said source and possibly the second aqueous composition originating from the means B are added to said culture medium so as to lower the pH, preferably until the pH of said culture medium reaches a lower limit, pH_{B};
∘ when the pH of said culture medium reaches said lower limit, pH_{B}, the first aqueous composition originating from the means A is added to said culture medium so as to raise the pH, preferably until the pH of said culture medium reaches an intermediate value, pH_{I}, with pH_{B}< pH_{I}< pH_{H};
- when the CO₂ source does not produce CO₂:
∘ the first aqueous composition originating from the means A is added to said culture medium so as to supply dissolved carbon to said culture medium, resulting in an increase of the pH until preferably the pH of said culture medium reaches an intermediate value, pH_{I}, with pH_{B}< pH_{I}< pH_{H};
∘ when the pH of said culture medium reaches an upper limit, pH_{H}, the second aqueous composition originating from the means B is added to said culture medium so as to lower the pH, preferably until the pH of said culture medium reaches a lower limit, pH_{B}.
